# EUROPEAN PATENT APPLICATION

(11) **EP 3 514 535 A1**
(43) Date of publication of application: **24.07.2019**
(21) Application number: 17850340.5
(22) Date of filing: 14.09.2017
(51) Int. Cl.: G01N 33/14, G01N 1/10, C12G 1/02, G01N 1/14

(54) **SYSTEM FOR CONTROLLING THE MALOLACTIC FERMENTATION OF WINES**

(30) Priority: 16.09.2016 ES 201631205
(71) Applicant: Inbiolev, S.L., 31195 Aizoain (ES)
(72) Inventor: GARCIA YOLDI, David, 31195 Aizoain (ES)
(86) International application number: PCT/ES2017/070610
(87) International publication number: WO 2018/050940

(57) **Abstract**

The system is provided for aspirating and suctioning a minimum quantity of wine from a barrel (1) or fermentation tank for the analysis of the malic acid, and optionally, the lactic acid, acetic acid and histamines, and thereby gaining control of the development of the malolactic fermentation of the wine inside the barrel (1), without needing to manually take samples for analysis. The suctioning or aspiration occurs via a tube (4-4') which passes through the corresponding lid (2) of the barrel, with the insertion of a solenoid valve (3) in the tube (4-4') and of a peristaltic pump (5) in the external section of the tube (4'), which peristaltic pump is the component which carries out the minimal suctioning of the wine in order to send same to sensors (6) that analyse the malic acid or other components, with the signals being sent wirelessly (7) to a computer, to a mobile telephone or any other means of displaying and viewing the results.

## Description

### PURPOSE OF THE INVENTION

This invention is a system for controlling the malolactic fermentation of wine, based on suction-vacuuming wine from the barrel or tank in which the fermentation of the wine is taking place. This takes place by way of a pump controlled by an electrovalve. The pump, which is peristaltic, has programmed timings which are coordinated with the electrovalve to conduct the suction-vacuuming in a controlled way, in time and volume. All of this is done so that once the vacuuming-suction is completed, a minimal amount of vacuumed wine passes through sensors that analyse the malic acid of the wine as well as, optionally, lactic and acetic acid and histamines.

The purpose of the invention is to provide the wine sector in general with a system that makes it possible to control the evolution of the malolactic fermentation of wine without needing to gather samples manually and analyse them in situ.

### BACKGROUND OF THE INVENTION

The fermentation of wine in barrels and/or fermentation tanks, such as those made out of stainless steel, concrete vats, etc., requires constant monitoring to understand the evolution of the malolactic fermentation at all times.

The current way of monitoring the fermentation in barrels and tanks involves extracting successive samples, a process which is performed continuously during the malolactic fermentation process, entailing a high cost in terms of staff time, as well as a high analytical cost and uncertainty most of the time due to samples not being taken frequently enough. This uncertainty means that the wine could possibly complete the malolactic fermentation without the winery being aware this due to not having taken the appropriate sample, which is not recommendable as ideally the wine is protected after the malolactic fermentation to prevent potential oxidation.

With the suggested system, which automatically extracts samples, we avoid that uncertainty or omission, and we provide guarantees for the process.

### DESCRIPTION OF THE INVENTION

This control system has been created to solve the aforementioned issue with a simple yet highly efficient solution, based on carrying out an automatic suction or vacuuming of the wine, once the alcoholic fermentation has been completed, by way of a time-programmed peristaltic pump in coordination with an electrovalve.

More specifically, the system is based on the insertion of a tube that passes through the lid on top of the barrel or fermentation tank. That tube also has an electrovalve attached. A stretch of the tube passes through the lid and into the barrel or tank, while the other end is outside, where the electrovalve and peristaltic suction pump are located. The corresponding sensors are positioned after this peristaltic pump in order to analyse mainly malic acid, but also lactic and acetic acid and histamines if required. All this is done in such a way that the signal sent by these sensors is wirelessly transmitted to a mobile phone, computer or similar device, in order for the person monitoring it to see this information.

In this way, the evolution of the malolactic fermentation can be monitored without having to extract samples in person for analysis.

Therefore, with this invention, the lid of the barrel or fermentation tank can be considered a "smart lid", a lid which should preferably be made out of silicone in the case of barrels, to allow for a transversal and central perforation, through which the aforementioned tube passes, a tube which is sealed with septum.

Between the lid and the peristaltic pump there is an electrovalve which will be programmed to work in coordination with the pump, ensuring the suction-vacuuming tube opens only when the extraction of the wine sample is going to take place.

The electrovalve will be preferably placed on top of the lid and outside the barrel, so that if it needs cleaning or replacing, these tasks can be done more easily.

The pump will suction a minimal amount of wine, always the same amount, while the peristaltic pump can also be actioned in reverse, so that once the sample has been analysed, the wine can be sent back down into the barrel or tank, ensuring the system is clean and free of liquid and thus also not affecting subsequent measurements.

When the monitoring of the fermentation is conducted in stainless steel or any other type of tank instead of barrels, these tanks will have an orifice or opening that allows the insertion of the tube into the tank in order to take samples.

Fermentation tanks generally have a tap or spigot in order to take samples, which can alternatively be used to insert the extraction tube. However, it is usually inserted in the bottom part of the tank, which is dangerous in the event of the poor functioning of the electrovalve which could lead to the tank not being sealed.

### DESCRIPTION OF THE DRAWINGS

In order to complement the following description and help achieve a better understanding of the invention's features, a drawing is provided as an integral part of a practical example in which at least the following has been represented:

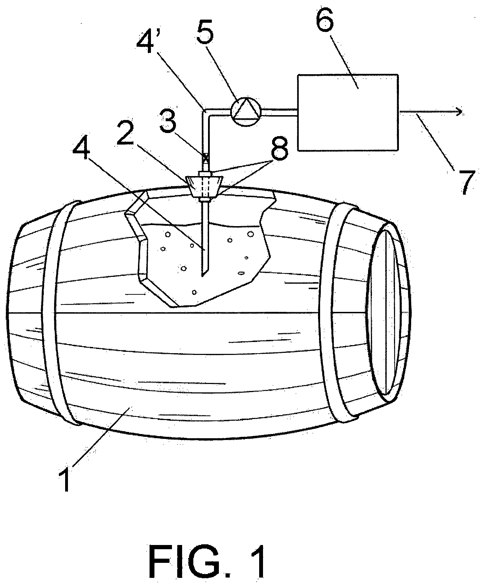

Figure 1. Corresponds to a schematic representation of a barrel of wine with the system for controlling the malolactic fermentation created in accordance with the purpose of the invention.

### PREFERENTIAL EMBODIMENT OF THE INVENTION

As can be seen in the aforementioned figure, the invention system, applicable as a system for controlling the fermentation of wine in barrels (1), is characterised by inserting a tube (4-4') into the lid (2), preferably made of silicone and located in the relevant orifice at the top of the barrel (1). This tube has a stretch (4) which reaches the inside the barrel (1), and more specifically into the wine inside, while the tube also has a stretch outside (4') which has an electrovalve attached (3) to control the opening and/or closing of the tube (4-4') and a peristaltic pump (5), which is in charge of sucking or vacuuming a sample of the wine from inside the barrel (1), sending it to an analyser or set of sensors (6) which can include a sensor to analyse the malic acid or fermentation of the wine, or other sensors to analyse lactic or acetic acid, or histamines. In this way, in any case, the wine is sent back to the barrel after being analysed (1), by actioning the peristaltic pump (5) in reverse.

The signals obtained in the analysis carried out by the relevant sensors (6) are sent wirelessly (7) to a mobile phone, computer, etc., where they can be easily seen and interpreted by the control staff.

Given all the aforementioned, the system described will make it possible to monitor the evolution of the malolactic fermentation of the wine located in a barrel or tank remotely, without requiring the available staff to take samples in order to analyse them, as is done traditionally. This is achieved thanks to the peristaltic pump (5), which is programmed and timed, and which carries out the vacuuming or sucking of a minimal amount of wine from inside the barrel (1) or tank, by way of a tube (4-4'), acting in coordination with the electrovalve (3), which is preferably placed above the appropriate lid (2) and outside the barrel or tank, in such a way that the tube (4-4') or extraction duct for taking samples only opens while the sucking and returning operations for each sample are taking place, closing afterwards to prevent the entry of oxygen inside the barrel or tank.

When monitoring of the wine's fermentation takes place in a stainless steel tank, or one of any other material, it will have the corresponding entrance to insert the tube (4-4') to take samples, an entrance preferably consisting of a threaded opening located on the top part of the tank itself to which to attach the lid (2), which will include the tube (4-4') passing through it for the suction, along with the inserted electrovalve (3) and peristaltic pump (5).

The peristaltic pump (5) can be used to pump in both directions, so that after the suctioned wine sample has been analysed, it is returned once again to the tank or barrel (1), in order to achieve the complete cleaning of the system before extracting the next sample.

Lastly, it must be noted that the tube (4-4') has, on the top and bottom ends with respect to the lid (2), seals (8) to prevent the entry of air.

## Claims

1. System for controlling the malolactic fermentation of wines, created to be applied both in barrels (1) that contain wine and where the fermentation takes place, as well as in stainless steel tanks or those of any other material where fermentation also takes place, which have a lid (2) and are **characterised by** this lid (2) including a tube that passes through it, leaving a stretch of tube (4) inside the tank or barrel (1) that contains the fermenting wine, and a stretch (4') outside the container; this latter stretch will have an electrovalve (3) and peristaltic pump (5) attached, which will be programmed and timed to suction a minimal amount of wine; with the peculiarity that this peristaltic pump (5) is linked to a set of sensors (6) for the analysis of malic acid and, optionally, lactic and acetic acid, and histamines.

2. System for controlling the malolactic fermentation of wines, as mentioned in claim 1, **characterised by** the lid (2), which is located in the top part of the tank or barrel (1), being made of silicone, and the tube (4-4') being a tube sealed with septum.

3. System for controlling the malolactic fermentation of wines, as mentioned in claims 1 and 2, **characterised by** the peristaltic pump (5) being bidirectional so that after the suctioned wine sample has been analysed, it can be sent back to the tank or barrel (1) in order to achieve a complete cleaning of the system before extracting the next sample.

4. System for controlling the malolactic fermentation of wines, as mentioned in the previous claims, **characterised by** the signals obtained in the process of analysis carried out by the sensors (6) being sent wirelessly (7) to a mobile phone, computer or similar device, to visualise the results.

5. System for controlling the malolactic fermentation of wines, as mentioned in claim 1, **characterised by** the tube (4-4') and the attached electrovalve (3) being inserted through the top part of a stainless steel tank.
